(19) Europäisches Patentamt

European Patent Office

Office européen des brevets

(11) EP 0 665 953 B1

(12) EUROPEAN PATENT SPECIFICATION

(45) Date of publication and mention
of the grant of the patent:
**21.01.1998 Bulletin 1998/04**

(21) Application number: **94900302.4**

(22) Date of filing: **25.10.1993**

(51) Int Cl.$^6$: **G01N 33/22**

(86) International application number:
**PCT/NL93/00213**

(87) International publication number:
**WO 94/10566 (11.05.1994 Gazette 1994/11)**

(54) **METHOD FOR DETERMINING THE CALORIFIC VALUE OF A GAS AND/OR THE WOBBE INDEX OF NATURAL GAS**

VERFAHREN ZUR BESTIMMUNG DES BRENNWERTES EINES GASES UND/ODER DER WOBBEZAHL EINES ERDGASES

PROCEDE DE DETERMINATION DE LA VALEUR CALORIFIQUE D'UN GAS ET/OU DE L'INDICE DE WOBBE DU GAZ NATUREL

(84) Designated Contracting States:
**AT BE CH DE DK ES FR GB GR IE IT LI LU MC NL PT SE**

(30) Priority: **23.10.1992 NL 9201845**

(43) Date of publication of application:
**09.08.1995 Bulletin 1995/32**

(73) Proprietor: **GASTEC N.V.**
**NL-7327 AC Apeldoorn (NL)**

(72) Inventor: **HÖRNEMANN, Johan, Adrianus, Tilmann**
**NL-7326 GG Apeldoorn (NL)**

(74) Representative:
**Smulders, Theodorus A.H.J., Ir. et al**
**Vereenigde Octrooibureaux**
**Nieuwe Parklaan 97**
**2587 BN 's-Gravenhage (NL)**

(56) References cited:
EP-A- 0 231 073       EP-A- 0 304 266
EP-A- 0 445 861       DE-A- 506 213
FR-A- 1 471 088       US-A- 4 020 480

## Description

The present invention relates to a method for determining the calorific value of a combustible gas. The invention further relates to a method for determining the Wobbe index of natural gas.

The determination of the calorific value of combustible gasses, such as natural gas and other fuel gasses, can be of importance for various reasons.

It is a fact that when natural gas from different sources is used, the composition, and therefore the calorific value, of the various gasses is not the same. For a buyer it may be of importance to know the calorific value so as to compensate fluctuations in conditions of use. Also, it is common for the price of the gas to be related to its calorific value.

The calorific value of a gas can be determined by burning the gas under conditions whereby the heat of combustion is measured. From the various data of the gas sample, such as mass, heat content, rise in temperature and the like, the calorific value can be accurately calculated. Such a method, however, is cumbersome and time-consuming, and therefore not suitable for the rapid in situ determination of the calorific value of the gas.

Accordingly, there is a need for a method which enables the calorific value of a combustible gas to be determined rapidly and accurately.

As regards the Wobbe index, too, it is important that it can be determined rapidly and accurately.

The invention accordingly relates to a method for determining the calorific value of a combustible gas, which method is characterized in that an accurately determined amount of gas is passed through a detection device, the signal obtained is integrated, the value thus obtained is compared with a calibration line and the calorific value is calculated therefrom.

The invention further relates to a method for determining the Wobbe index of natural gas, which method is characterized in that an accurately determined amount of natural gas is passed through a detection device, the signal obtained is integrated, the value thus obtained is compared with a calibration line and the Wobbe index is calculated from the calorific value thus obtained, combined with the density of the gas.

As a detection device, use is preferably made of a hydrocarbon detector based on catalytic combustion, more particularly a methane detector. Such detectors are commercially available and comprise *inter alia* a combustion chamber in which a temperature-sensitive resistance wire is located having applied thereto a catalyst for the catalytic combustion of hydrocarbons. If this wire comes into contact with a combustible gas, a combustion occurs whereby the resistance of the wire changes. This change can be ascertained, for instance with a Wheatstone bridge.

Surprisingly, it has now been found that it is possible to pass a known amount of gas through such a detection device and to obtain a reliable value for the calorific value from the measuring result. This is particularly unexpected since such detectors are not based on combustion of the total amount of gas but only on a part thereof. It has nonetheless been found that the signal of such a detector can be used for obtaining a reliable measuring result.

Accordingly, if an exactly known amount of combustible gas, such as natural gas, is passed through a hydrocarbon detector, the result obtained after integration of the signal, i.e., after determination of the area under the curve, is a value which upon comparison with a calibration line accurately indicates the calorific value of the gas.

In the case where the Wobbe index of natural gas is to be determined, the determination of the calorific value as described above can be combined with a determination of the density of the natural gas, for instance with a katharometer. When using a katharometer for determining the density of the gas, the heat conductivity of the gas is determined with this meter. This quantity can subsequently be converted to the density of the gas, for instance with the aid of a calibration line. The determination can be performed on the same sample stream as that of which the calorific value is determined. This can for instance occur in parallel, or prior to the determination of the calorific value. The Wobbe index is obtained from the thus obtained data for the calorific value and the density.

An important advantage of the method according to the invention is the simplicity, speed and accuracy with which the determinations can be carried out. It is possible to carry out a determination within a few tens of seconds. This can be of great importance, in particular for process control or large scale consumption of gas. The accuracy of the determination of the calorific value appears to be very good; the error is less than 0.05 %.

According to the invention, it is for instance possible to allow the gas whose calorific value is to be determined to flow from a mainstream through a sampling conduit, whereafter the sampling conduit is shut off from the mainstream and is brought into communication with a sampling stream and the contents of the sampling conduit is passed entirely through the detection device. If desired, the gas can be diluted. The method according to the invention can simply be carried out with the aid of a plurality of cocks, for instance two four-way cocks which are connected as described in the drawing. Naturally, it is also possible to utilize other designs, for instance starting from two, three or six-way cocks. Such a system can be advantageously controlled with the aid of a computer which provides not only for the control of the apparatus but also for the calculation of the calorific value and/or the Wobbe index.

Other methods where an exact amount of gas is supplied to a measuring device are also applicable. For instance, use could be made of a system based on pulse techniques. Thus, it is also possible to supply the gas to the detector pulse-wise. In that case, a sine-shaped measured signal can be obtained, whose amplitude is a measure of a calorific value.

The present invention can be used for determining the calorific value of various types of combustible gas. Examples include natural gas, synthesis gas, fuel gas, refinery gas and pyrolysis gas.

The invention also relates to an apparatus for determining the calorific value of a combustible gas, comprising a hydrocarbon detector, means for supplying an accurately determined amount of gas to the detector, means for determining the signal of the detector, means for integrating the signal thus determined and means for comparing the integrated signal with calibration values and calculating the calorific value of the gas.

An apparatus for determining the Wobbe index of natural gas comprises the same components as the apparatus for determining the calorific value of a gas, with means added thereto for determining the density of the natural gas and means for calculating the Wobbe index from the density and the calorific value.

The invention will now be elucidated with reference to some drawings.

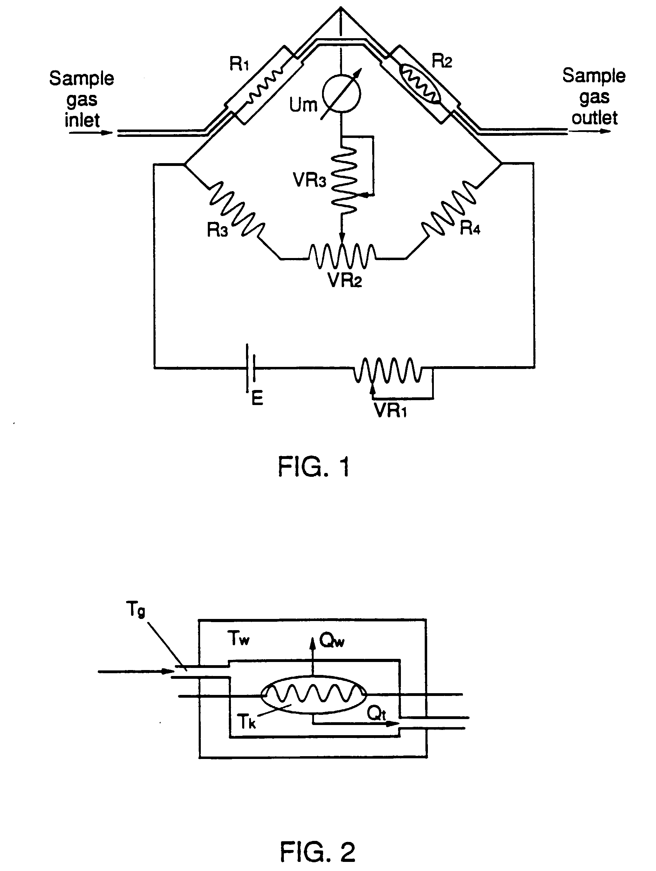

In Fig. 1 the principle of the measurement is elucidated.

The sample gas flows through two measuring cells in which filaments $R_1$ en $R_2$ are arranged. Filament $R_2$ is provided with a catalyzing substance. If a combustible gas is present in the sample air passing through, the heat production of $R_2$, as a result of the catalytic combustion, will be greater than at $R_1$. As a result of the additionally produced heat, the temperature of $R_2$ and therefore the electric resistance of $R_2$ becomes higher than that of $R_1$.

The electric equilibrium of the bridge circuit is removed and the resultant measured signal $U_m$ is a measure for the additionally produced heat of $R_2$.

The temperature of the filaments and the catalyst is set with $Vr_1$. With $Vr_2$ the zero point is set. With $Vr_3$ the span is set.

The reference cell and the catalytic measuring cell are thermally coupled. Both cells are accommodated in a solid thermally inert measuring block. As a result, variations in ambient temperature and the temperature of the sample air have only a minimum influence on the measured signal.

Fig. 2 schematically shows a possible measuring cell. In stationary condition, with a fixed air/gas ratio, the produced heat of the filament will be removed partly to the cell wall and partly by the air stream.

$$Qf = C_1(T_k\text{-}T_g) \qquad Q_w = C_2(T_k\text{-}T_w)$$

$$Q1 = Qj + Q_w = C_1(T_k\text{-}T_g) + C_2(T_k\text{-}T_w)$$

$$= C_1 T_k - C_1 T_g + C_2 T_k - C_2 T_w$$

$$U_m = C_m \times Q1 = (C_1 + C_2)T_k - C_1 T_g - C_2 T_w$$

$$U_m = C_m \{(C_1 + C_2)T_k - C_1 T_g - C_2 T_w\}$$

In case of a small modification of the air/gas ratio or the gas composition, first a modification of Tk will arise.

$$\Delta U_m = C_m \{(C_1 + C_2)\Delta T_n - C_1 T_g - C_r T_w\}$$

Owing to the thermal inertia of the measuring block, $T_w$ will adjust after some time to a new thermal equilibrium. The consequence is that $U_m$ will achieve an equilibrium value only after some time has passed.

The temperature of the air supplied also affects $U_m$. If a small amount of the gas to be measured is introduced into the air stream of the measuring cell, $T_w$ will hardly change during the passage of the gas owing to the thermal inertia. The measured signal is not subject to the influence of this inertia.

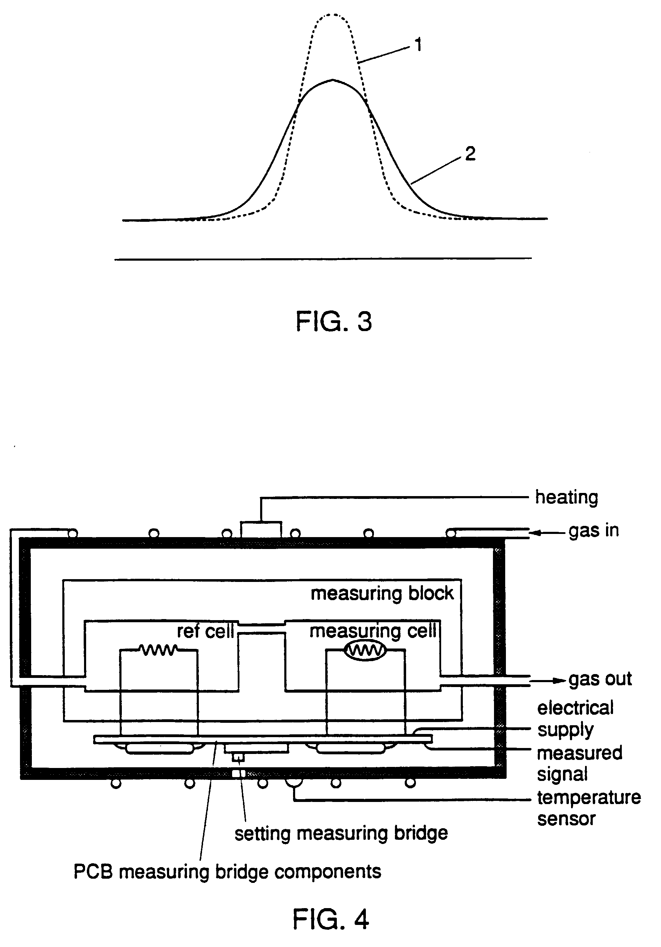

When a sample loop is used, the measured signal has the shape of a Gaussian curve. The area of the curve is proportional to the energy generated by the catalytic combustion. Modification of the air stream results in the curve becoming more or less sharp (curves 1 and 2 of Fig. 3). The area of the Gaussian curve, however, remains constant.

The integrated value of the measured signal always gives a good value for the generated energy of the combusted amount of gas. When a single measuring cell is used, the maximum of the curve will be affected by $T_g$ and $T_w$. By using a non-catalytically active reference cell, connected in a bridge circuit, these effects are reduced to an important extent. Because geometrically the two measuring cells are not completely identical, it may be necessary in connection with the accuracy and the reproducibility of the measuring system to accommodate the measuring block in a thermostated box, the sample gas, before entering the measuring cells, flowing through a heat exchanger which is thermally coupled with the box. The temperature of this box must be constant, for instance a few degrees of the maximum ambient temperature.

A possible embodiment is shown in Fig. 4.

This figure shows a thermostated box in which, to improve the measuring accuracy and the stability, the measuring block including the two measuring cells and the components together constituting the electronic measuring bridge are accommodated. The wall temperature of the box is electronically controlled to a temperature approximately 10 degrees above the maximum ambient temperature. The electric current for the measuring bridge is supplied by an electronically stabilized supply.

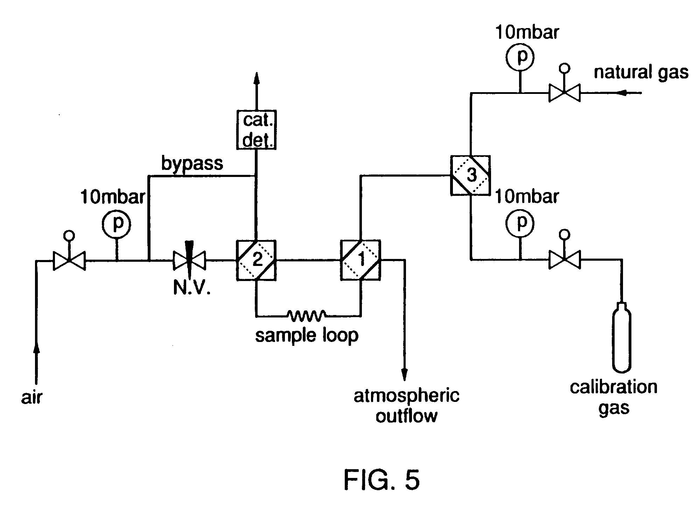

Fig. 5 shows a possible embodiment of the calorimeter according to the invention. The system is made up of three functional elements, viz. the sample selection, the sample taking and the bypass system.

For calibration a possibility of sample selection is built in. By adding switching valves, various calibration gasses can be connected to the measuring system.

The sampling system comprises two four-way cocks, nos. 1 and 2 and a sample loop. With this system it is possible to take an accurately reproducible amount of the gas to be examined. A first condition for this is an atmospheric outflow during flushing and a sample loop which is not unduly narrow, there being sufficient throughflow at a sample gas prepressure of approx. 10 mbar. A second condition is that the cocks 1 and 2 are consecutively switched with a short interval. By switching cock 1, the sample gas is isolated in the sample loop. By switching cock 2, this isolated amount of gas is passed via the bypass system into the supply conduit of the catalytic detector.

With this flow system, the influence of gas, prepressure and volume of the sample loop appear not to be critical for the measuring accuracy.

The bypass system controls the mixing of the gas to be measured with air. The concentration of gas, which should preferably not exceed the maximum limit of the detector, which is generally 5%, is controlled by the needle valve.

The required air can be supplied from a pressure cylinder. Because the prepressure is low, for instance approx. 10 mbar, it is also possible to provide the required air by including a pump in the discharge conduit of the catalytic detector, with ambient air being used. In that case, the supply conduit of the bypass system can optionally be provided with an active carbon filter to filter out any hydrocarbons and other combustible components present in the outside air.

In combination with this system, preferably a computer is used for processing the data into the calorific top value of the gas. With this computer, however, it is also possible to provide for the control of the entire sample taking.

In the case where the apparatus according to the invention is to be used for determining the Wobbe index of natural gas, in addition to the calorific top value ($H_s$), the density of the gas must be known as well.

$$WI = \frac{H_s}{\sqrt{d}}$$

If a suitable mass flow sensor is included in the supply conduit of the catalytic detector, the measuring system can also determine the Wobbe index. It is also possible to arrange this mass flow sensor in parallel with the calorimeter.

The signal of the mass flow sensor also has the shape of a Gaussian curve. The integral of this signal $S_m$ is a measure of the mass of the gas in the air sample.

$$d = C \int_{T_1}^{T_2} S_m \, dt - background$$

## Claims

1. A method for determining the calorific value of a combustible gas, characterized in that an accurately determined amount of gas is passed through a hydrocarbon detection device, the signal obtained is integrated, the value thus obtained is compared with calibration values and the calorific value is calculated therefrom.

2. A method for determining the Wobbe index of natural gas, characterized in that an accurately determined amount of gas is passed through a hydrocarbon detection device, the signal obtained is integrated, the value thus obtained is compared with calibration values, the calorific value is calculated therefrom and the Wobbe index is determined from this calorific value and the measured density of the natural gas.

3. A method according to claim 2, characterized in that the density of the gas is determined with the aid of a katharometer.

4. A method according to claims 1-3, characterized in that, as a detection device, use is made of a hydrocarbon detector based on catalytic combustion, or a flame ionization detector.

5. A method according to claims 1-2, characterized in that the combustible gas of which the calorific value is determined is natural gas, synthesis gas, fuel gas, refinery gas or pyrolysis gas is determined.

6. A method according to claims 1-5, characterized in that the gas whose calorific value and/or Wobbe index is to be determined, is allowed to flow from a mainstream through a sampling conduit, the sampling conduit is shut off from the mainstream and brought into communication with a sampling stream and the contents of the sampling conduit is passed through at least one detection device.

7. Apparatus for determining the calorific value of a combustible gas utilizing the method according to any one of claims 1-6, comprising means for accurately taking a sample, means for detecting a property of the gas, which property is proportional to the calorific value thereof, means for integrating the thus obtained signal and means for comparing the integrated signal with calibration values and means for calculating the calorific value therefrom.

8. Apparatus for determining the Wobbe index of natural gas utilizing the method according to any one of claims 2-6, comprising means for accurately taking a sample, means for detecting a property of the gas, which property is proportional to the calorific value thereof, means for integrating the thus obtained signal, means for comparing the integrated signal with calibration values, means for determining the density of the natural gas and means for calculating the Wobbe index from the density and the calorific value.

9. Apparatus according to claim 8, characterized in that, as a device for determining the density of the gas, a katharometer is used.

**Patentansprüche**

1. Verfahren zur Bestimmung des Brennwertes eines brennbaren Gases, dadurch gekennzeichnet, daß eine genau bestimmte Gasmenge durch eine Kohlenwasserstoffdetektionsvorrichtung geleitet, das erhaltene Signal integriert, der so erhaltene Wert mit den Kalibrierungswerten verglichen und daraus der Brennwert errechnet wird.

2. Verfahren zur Bestimmung der Wobbezahl von Erdgas, dadurch gekennzeichnet, daß eine genau bestimmte Gasmenge durch eine Kohlenwasserstoffdetektionsvorrichtung geleitet, das erhaltene Signal integriert, der so erhaltene Wert mit Kalibrierungswerten verglichen, daraus der Brennwert errechnet und die Wobbezahl aus dem Brennwert und der gemessenen Dichte des Erdgases berechnet wird.

3. Verfahren nach Anspruch 2, dadurch gekennzeichnet, daß die Dichte des Gases mit Hilfe eines Katharometers bestimmt wird.

4. Verfahren nach den Ansprüchen 1-3, dadurch gekennzeichnet, daß als Detektionsvorrichtung ein Kohlenwasserstoffdetektor, der auf katalytischer Verbrennung beruht, oder ein Flammenionisationsdetektor verwendet wird.

5. Verfahren nach den Ansprüchen 1-2, dadurch gekennzeichnet, daß das brennbare Gas, dessen Brennwert bestimmt wird, Erdgas, Synthesegas, Brenngas, Raffineriegas oder Pyrolysegas ist.

6. Verfahren nach den Ansprüchen 1-5, dadurch gekennzeichnet, daß es dem Gas, dessen Brennwert und/oder Wobbezahl bestimmt wird, ermöglicht wird, von einem Hauptstrom durch eine Probeleitung zu fließen, die Probeleitung von dem Hauptstrom abgesperrt und mit einem Probstrom in Verbindung gebracht wird, und der Inhalt der Probeleitung durch wenigstens eine Detektionsvorrichtung geleitet wird.

7. Vorrichtung zur Bestimmung des Brennwertes eines brennbaren Gases unter Anwendung des Verfahrens nach einem der Ansprüche 1-6, mit einer Vorrichtung zur genauen Entnahme einer Probe, einer Vorrichtung zur Detektion einer Eigenschaft des Gases, die sich proportional zum Brennwert verhält, einer Vorrichtung zur Integration des so erhaltenen Signals, einer Vorrichtung zum Vergleichen des integrierten Signals mit Kalibrierungswerten und einer Vorrichtung zur Berechnung des Brennwertes aus diesen Werten.

8. Vorrichtung zur Bestimmung der Wobbezahl von Erdgas unter Anwendung des Verfahrens nach einem der Ansprüche 2-6, mit einer Vorrichtung zur genauen Entnahme einer Probe, einer Vorrichtung zur Detektion einer Eigenschaft des Gases, die sich proportional zum Brennwert verhält, einer Vorrichtung zur Integration des so erhaltenen Signals, einer Vorrichtung zum Vergleichen des integrierten Signals mit Kalibrierungswerten, einer Vorrichtung zur Bestimmung der Dichte des Erdgases und einer Vorrichtung zur Berechnung der Wobbezahl aus der Dichte und dem Brennwert.

9. Vorrichtung nach Anspruch 8, dadurch gekennzeichnet, daß als Vorrichtung zur Bestimmung der Dichte des Gases ein Katharometer verwendet wird.

**Revendications**

1. Procédé de détermination du pouvoir calorifique d'un gaz combustible, caractérisé en ce qu'une quantité de gaz déterminée avec précision circule dans un dispositif de détection d'hydrocarbures, le signal obtenu est intégré, la valeur obtenue est comparée à des valeurs d'étalonnage et le pouvoir calorifique est calculé en conséquence.

2. Procédé de détermination de l'indice Wobbe du gaz naturel, caractérisé en ce qu'une quantité de gaz déterminée avec précision circule dans un dispositif de détection d'hydrocarbures, le signal obtenu est intégré, la valeur ainsi obtenue est comparée à des valeurs d'étalonnage, le pouvoir calorifique est calculé en conséquence, et l'indice Wobbe est déterminé d'après ce pouvoir calorifique et la masse volumique mesurée du gaz naturel.

3. Procédé selon la revendication 2, caractérisé en ce que la masse volumique du gaz est déterminée à l'aide d'un catharomètre.

4. Procédé selon les revendications 1 à 3, caractérisé en ce qu'il comprend l'utilisation, comme dispositif de détection, d'un détecteur d'hydrocarbures mettant en oeuvre une combustion catalytique ou un détecteur à ionisation de flamme.

5. Procédé selon les revendications 1 et 2, caractérisé en ce que le gaz combustible dont le pouvoir calorifique est déterminé est le gaz naturel, un gaz de synthèse, un gaz combustible, un gaz de raffinerie ou un gaz de pyrolyse.

6. Procédé selon les revendications 1 à 5, caractérisé en ce que le gaz dont le pouvoir calorifique et/ou l'indice Wobbe doivent être déterminés peut s'écouler à partir d'un courant principal vers un conduit d'échantillonnage, le conduit d'échantillonnage est séparé du courant principal et est mis en communication avec un courant d'échantillonnage, et le contenu du conduit d'échantillonnage est transmis par au moins un dispositif de détection.

7. Appareil de détermination du pouvoir calorifique d'un gaz combustible par mise en oeuvre du procédé selon l'une quelconque des revendications 1 à 6, comprenant un dispositif de prise précise d'un échantillon, et un dispositif de détection d'une propriété du gaz, cette propriété étant proportionnelle à son pouvoir calorifique, un dispositif d'intégration du signal ainsi obtenu, et un dispositif de comparaison du signal intégré à des valeurs d'étalonnage et un dispositif de calcul du pouvoir calorifique en conséquence.

8. Appareil de détermination de l'indice Wobbe du gaz naturel par mise en oeuvre du procédé selon l'une quelconque des revendications 2 à 6, comprenant un dispositif de prise précise d'un échantillon, un dispositif de détection d'une propriété du gaz, cette propriété étant proportionnelle au pouvoir calorifique du gaz, un dispositif d'intégration du signal ainsi obtenu, un dispositif de comparaison du signal intégré à des valeurs d'étalonnage, un dispositif de détermination de la masse volumique du gaz naturel, et un dispositif de calcul de l'indice Wobbe à partir de la masse volumique et du pouvoir calorifique.

9. Appareil selon la revendication 8, caractérisé en ce qu'on utilise, comme dispositif de détermination de la masse volumique du gaz, un catharomètre.

FIG. 1

FIG. 2

FIG. 3

FIG. 4

FIG. 5